# EUROPÄISCHE PATENTSCHRIFT

(11) **EP 0 682 018 B1**
(45) Veröffentlichungstag und Bekanntmachung des Hinweises auf die Patenterteilung: **20.05.1998**
(21) Anmeldenummer: 95106196.9
(22) Anmeldetag: 26.04.1995
(51) Int. Cl.: C07D 239/42

(54) **Verfahren zur Herstellung von 2-Amino-4,6-dichloro-pyrimidin**
Process for the preparation of 2-amino-4,6-dichloropyrimidine
Procédé pour la préparation de 2-amino-4,6-dichloropyrimidine

(30) Priorität: 13.05.1994 AT 995/94
(43) Veröffentlichungstag der Anmeldung: 15.11.1995
(73) Patentinhaber: DSM Chemie Linz GmbH, 4021 Linz (AT)
(72) Erfinder: Schwarz, Franz-Thomas, Dipl.-Ing., A-4493 Wolfern (AT); Altreiter, Johann, A-4212 Neumarkt (AT)
(74) Vertreter: Kunz, Ekkehard, Dr.

(56) Entgegenhaltungen:
- WO-A-95/07265
- US-A- 3 991 190
- US-A- 4 929 729

## Beschreibung

2-Amino-4,6-dichlorpyrimidin (ADCP) ist ein bekanntes und wertvolles Zwischenprodukt für Medikamente und Agrarchemikalien.

Aus der Literatur sind daher bereits einige Verfahren zur Herstellung von ADCP bekannt. So ist beispielsweise in US 3,991,190, Beispiel 1, ein Verfahren beschrieben, bei dem 2-Amino-4,6-dihydroxipyrimidin (ADHP) mit einem Überschuß an Phosphoroxichlorid und Dimethylanilin als Säurefänger unter Rückflußkochen bei einer Temperatur von etwa 107°C zu ADCP umgesetzt wird. Die Isolierung von ADCP erfolgt dabei nach Abdestillieren von überschüssigem Phosphoroxichorid, durch Suspendieren in Wasser und Zugabe von Natronlauge, bis ein pH-Wert von 8 bis 9 erreicht ist. Bei diesem Verfahren entsteht jedoch eine Vielzahl von Nebenprodukten und die Ausbeute an ADCP ist relativ gering (70 %). Als Verbesserungsvorschlag wird in US 4,929,729 die Umsetzung von ADHP mit Phosphoroxychlorid bei Temperaturen von 50 - 100°C in Gegenwart eines Säurefängers in einem zusätzlichen Lösungsmittel angeführt. Die Ausbeute an ADCP liegt im Vergleich zu US 3,991,190 um etwa 10 % höher (82 - 85 %), wobei die Reinheit etwa 91 - 95 % beträgt. Der Nachteil dieses Verfahrens ist jedoch die Notwendigkeit eines zusätzlichen Lösungsmittels.
Ein weiterer Nachteil ist, daß, wie in den Beispielen von US 4,929,729 durchgeführt, zur Isolierung von ADCP das Reaktionsgemisch, nach erfolgter Chlorierung und Abtrennung von überschüssigem Phosphoroxychlorid und dem Lösungsmittel, auf Eiswasser gegossen und anschließend wieder auf etwa 50°C erwärmt werden muß, wofür ein zusätzlicher Energieaufwand nötig ist.

Aufgabe der vorliegenden Erfindung war es demnach ein Verfahren zur Herstellung von 2-Amino-4,6-dichlorpyrimidin zu finden, das ohne zusätzliches Lösungsmittel zu ADCP in gegenüber dem Stand der Technik höherer Ausbeute und höherer Reinheit führt, die Bildung von Nebenprodukten verhindert und den zusätzlichen Energieaufwand bei der Isolierung von ADCP vermeidet.

Unerwarteterweise konnte diese Aufgabe durch das erfindungsgemäße Verfahren gelöst werden.

Gegenstand der vorliegenden Erfindung ist daher ein Verfahren zur Herstellung von 2-Amino-4,6-dichlorpyrimidin, das dadurch gekennzeichnet ist, daß 2-Amino-4,6-dihydroxipyrimidin bei einer Temperatur von 20 bis 80°C in Gegenwart von Triethylamin als Säurefänger mit Phosphoroxichlorid, in einem molaren Verhältnis von 2-Amino-4,6-dihydroxipyrimidin zu Phosphoroxichlorid von über 1 : 5 bis zu 1 : 8, umgesetzt wird und 2-Amino-4,6-dichlorpyrimidin isoliert wird.
Bei dem erfindungsgemäßen Verfahren wird 2-Amino-4,6-dihydroxipyrimidin (ADHP) mit einem Überschuß an POCl₃ zu 2-Amino-4,6-dichloropyrimidin (ADCP) umgesetzt.
ADHP wird dazu in POCl₃ suspendiert, das sowohl als Reagens als auch als Reaktionsmedium dient, wodurch kein zusätzliches Lösungs- bzw. Verdünnungsmittel nötig ist. Durch die eingesetzte Menge an POCl₃ wird die Handhabbarkeit der Suspension bestimmt. Das molare Verhältnis von ADHP zu POCl₃ kann dabei variieren, wobei die Untergrenze durch die Rührbarkeit der Suspension und die Obergrenze hauptsächlich durch wirtschaftliche Faktoren beeinflußt werden.
Bevorzugt wird ein molares Verhältnis von ADHP zu POCl₃ bis zu 1 : 6 eingesetzt.
Ein größerer Überschuß an POCl₃ hat keine nachteiligen Auswirkungen auf die Reaktion und kann gewünschtenfalls auch eingesetzt werden, ist jedoch aus wirtschaftlichen Gründen nicht sinnvoll.
Die so erhaltene Suspension wird dann auf eine Temperatur von 20 - 80°C erwärmt. Je dickflüssiger die zu erwärmende Suspension ist, desto höher sollte dabei die Temperatur liegen, um ausreichende Rührbarkeit und somit Durchmischung zu ermÖglichen. Bevorzugt wird die Suspension auf 40 bis 80°C, besonders bevorzugt auf 70 bis 75°C, erhitzt.

Anschließend wird Triethylamin in einer äquivalenten Menge über einen Zeitraum von etwa 20 bis 80 Minuten, bevorzugt von 30 bis 60 Minuten, zudosiert. Es kann aber auch ein leichter Überschuß an Triethylamin verwendet werden, sodaß das molare Verhältnis von ADHP zu Triethylamin etwa 1 : 2 bis 1 : 3, bevorzugt bis 1 : 2,25 beträgt.
Nach beendeter Dosierung wird noch etwa 2 bis 10 Minuten, bevorzugt 4 bis 6 Minuten bei der Reaktionstemperatur weiter gerührt. Zur Isolierung und Aufarbeitung von ADCP wird überschüssiges POCl₃ unter Vakuum abdestilliert. Die Sumpftemperatur kann dabei in Abhängigkeit vom erreichten Vakuum variieren, wobei die Untergrenze durch die Rührbarkeit des Reaktionsgemisches beeinflußt wird und die'Obergrenze bei etwa 80°C liegt. Bevorzugt wird die Sumpftemperatur auf etwa 70 bis 75°C gehalten. Der Rückstand wird bei einer Temperatur von etwa 40 bis 60°C in Wasser eingerührt. Die so erhaltene Suspension wird noch etwa 1,5 bis 2,5 Stunden bei etwa 40 bis 60°C gerührt und anschließend mit 20 bis 50 %iger, bevorzugt mit 20 %iger NaOH versetzt, bis ein pH-Wert von etwa 2,5 bis 4, bevorzugt von etwa 3 erreicht ist.

Zuletzt wird das ausgefallene Produkt abfiltriert, mit Wasser gewaschen und an der Luft getrocknet. Mit dem erfindungsgemäßen Verfahren wird ADCP in gegenüber dem Stand der Technik höheren Ausbeuten von über 90 % und in höherer Reinheit (ca. 99 %) erhalten.

### Beispiel 1

In einem 250 ml Doppelmantelglasreaktor mit Rührwerk, Rückflußkühler und Thermostatheizung wurden der Reihe nach 100 ml (1,12 Mol) Phosphoroxichlorid und 25,4 g (0,2 Mol) 2-Amino-4,6-dihydroxipyrimidin vorgelegt. Die so erhaltene Suspension wurde auf 75°C erwärmt und anschließend über einen Zeitraum von 1 Stunde 46 g (0,45 Mol) Triethylamin zudosiert. Nach beendeter Dosierung wurde noch 5 Minuten bei 75°C gerührt und anschließend überschüssiges Posphoroxichlorid unter Vakuum abdestilliert, wobei die Sumpftemperatur auf etwa 75°C gehalten wurde. Der Rückstand wurde bei einer Temperatur von etwa 50°C in 200 ml Wasser eingerührt und die so erhaltene Suspension noch 2 Stunden bei 50°C weitergerührt. Anschließend wurde die Suspension mit 20 %iger NaOH versetzt, bis ein pH-Wert von 3 erreicht war.
Das ausgefallene Produkt wurde abfiltriert, mit Wasser gewaschen und an der Luft getrocknet.
Ausbeute: 30,2 g (92,1 %)
Gehalt aus GC: 99 %

## Patentansprüche

1. Verfahren zur Herstellung von 2-Amino-4,6-dichlorpyrimidin, dadurch gekennzeichnet, daß 2-Amino-4,6-dihydroxipyrimidin bei einer Temperatur von 20 bis 80°C in Gegenwart von Triethylamin als Säurefänger mit Phosphoroxichlorid in einem molaren Verhältnis von 2-Amino-4,6-dihydroxipyrimidin zu Phosphoroxichlorid von über 1 : 5 bis zu 1 : 8 umgesetzt wird und 2-Amino-4,6-dichlorpyrimidin isoliert wird.

2. Verfahren nach Anspruch 1, dadurch gekennzeichnet, daß Triethylamin in einem molaren Verhältnis zu 2-Amino-4,6-dihydroxipyrimidin von 2 : 1 bis 3 : 1 eingesetzt wird.

3. Verfahren nach Anspruch 1, dadurch gekennzeichnet, daß zur Isolierung von 2-Amino-4,6-dichlorpyrimidin überschüssiges Phosphoroxichlorid unter Vakuum abdestilliert wird, der Rückstand in Wasser bei 40 - 60°C suspendiert wird, die so erhaltene Suspension nach 1,5 bis 2,5 Stunden Rühren mit 20 - 50 %iger NaOH versetzt wird bis ein pH-Wert von etwa 2,5 bis 4 erreicht ist, worauf das ausgefallene 2-Amino-4,6-dichlorpyrimidin abfiltriert, mit Wasser gewaschen und an der Luft getrocknet wird.

## Claims

1. Process for the preparation of 2-amino-4,6-dichloropyrimidine, characterized in that 2-amino-4,6-dihydroxypyrimidine is reacted with phosphorus oxychloride , in a molar ratio of 2-amino-4,6-dihydroxypyrimidine to phosphorus oxychloride of more than 1 : 5 up to 1 : 8,at a temperature of 20 to 80°C in the presence of triethylamine as the acid-trapping agent, and 2-amino-4,6-dichloropyrimidine is isolated.

2. Process according to Claim 1, characterized in that the triethylamine is employed in a molar ratio to the 2-amino-4,6-dihydroxypyrimidine of 2 : 1 to 3 : 1.

3. Process according to Claim 1, characterized in that, to isolate the 2-amino-4,6-dichloropyrimidine, excess phosphorus oxychloride is distilled off in vacuo, the residue is suspended in water at 40 - 60°C, after stirring for 1.5 to 2.5 hours, 20 - 50% strength NaOH is added to the suspension thus obtained until a pH of about 2.5 to 4 is reached, after which the 2-amino-4,6-dichloropyrimidine which has precipitated out is filtered off, washed with water and dried in air.

## Revendications

1. Procédé de préparation de 2-amino-4,6-dichloropyrimidine, caractérisé en ce qu'on fait réagir de la 2-amino-4,6-dihydroxypyrimidine à une température de 20 à 80°C, en présence de triéthylamine comme capteur d'acide, avec de l'oxychlorure de phosphore, selon un rapport molaire de la 2-amino-4,6-dihydroxypyrimidine à l'oxychlorure de phosphore de plus de 1 : 5 à 1 : 8, et on isole la 2-amino-4,6-dichloropyrimidine.

2. Procédé selon la revendication 1, caractérisé en ce qu'on utilise un rapport molaire de la triéthylamine à la 2-amino-4,6-dihydroxypyrimidine de 2 : 1 à 3 : 1.

3. Procédé selon la revendication 1, caractérisé en ce que, pour isoler la 2-amino-4,6-dichloropyrimidine, on élimine l'oxychlorure de phosphore en excès par distillation sous vide, on met le résidu en suspension dans de l'eau à une température de 40 à 60°C, après 1,5 à 2,5 heures, on ajoute du NaOH à 20 - 50 % à la suspension ainsi obtenue, sous agitation, jusqu'à l'obtention d'une valeur de pH d'environ 2,5 à 4, après quoi on filtre la 2-amino-4,6-dichloropyrimidine précipitée, on la lave avec de l'eau et on la sèche à l'air.
